# EUROPEAN PATENT APPLICATION

(11) **EP 2 351 587 A1**
(43) Date of publication of application: **03.08.2011**
(21) Application number: 10152305.8
(22) Date of filing: 01.02.2010
(51) Int. Cl.: A61M 1/00, A61M 3/02

(54) **Surgical irrigator**

(71) Applicant: Bidoia s.a.s. di Gianfranco Bidoia E C., I-35010 Vigonza (Padova) (IT)
(72) Inventor: Bidoia, Gianfranco, 35142, PADOVA (IT)
(74) Representative: Modiano, Micaela Nadia

(57) **Abstract**

A surgical irrigator (10) for washing body regions subjected to an operation, which has a containment casing (11) inside which there are means (12) for pumping a washing liquid that arrives from a tank which can be associated therewith and an electric motor (13) for actuating the pumping means; the electric motor is powered by the mains by means of:
- a power supply (14), with which there are associated
- at least one assembly (15, 16) for limiting the output voltage to a value (Vu1, Vu2) that is not higher than the nominal operating voltage (Vn) of the electric motor, and
- means (17) for switching the output voltage to a value (Vu3) that is higher than the nominal operating value (Vn) of the electric motor (13).

## Description

The present invention relates to a surgical irrigator for washing body regions subjected to an operation, particularly but not exclusively useful in orthopedic surgery.

During surgery it is often necessary to clean the regions on which one is operating in order to improve the surgeon's view, remove blood losses and remove other physiological residues.

The physiological residues can be portions of soft tissue, cartilages and bone fragments.

These cleaning operations sometimes require a certain "vigor" for removing the residues that have remained partially attached to the part from which they originated.

At the same time, the action of these cleaning operations can advantageously lead to limited hemostasis in the regions on which one operates.

The need for "energetic" cleaning actions occurs especially in orthopedic surgery, both in prosthetic operations and in osteosynthesis operations.

In this type of activity it is in fact normal to cut, drill, mill, correct, bore, file bone portions and accordingly obtain residues that have a hard consistency and often also have substantial dimensions.

These operations may further cause blood losses which must be eliminated or blocked, if not totally, at least partially.

Elimination of physiological residues and blood losses generally takes place by means of absorbing systems or by means of washes, which are followed by a step of aspirating the used washing liquid (in which the blood loss has been diluted and the physiological residues have been dispersed).

In order to perform homeostasis, the method of spraying with a certain pressure an aqueous liquid (optionally with the addition of substances adapted for the purpose) onto the regions with reduced bleeding is known among the various methods that are performed.

It is understood that the hemostasis effect appears to be more effective if the thrust of the liquid is intermittent ("pulsed"), particularly if the pause between successive thrusts is substantially equal to the duration of such thrusts.

The "pulsed" effect of the thrust of the washing liquid is also more effective to separate the physiological residues that have remained partially attached to their regions of origin.

Various devices are commercially available for performing these washing operations and they all use, in any case, the same functional particularities.

These devices (generally known as "irrigators"), which convey the sterile washing liquid to the region subjected to an operation, can also comprise integrated suction systems.

A particular and efficient type of these surgical irrigators is disclosed and claimed in EP 1799280 and EP 1977774 in the name of this same Applicant.

These European patent applications describe a surgical irrigator for washing body regions subjected to an operation, which has a containment casing inside which there are means for feeding the washing liquid that arrives from a tank which can be associated therewith; the containment casing is constituted substantially by two distinct portions, respectively a grip portion for the user's hand and a portion for the ejection of the washing liquid toward the region to be washed.

This irrigator is characterized in that it comprises means for the articulation of the ejection portion with respect to the grip portion, and in that it is powered by a transformer which is connected to the mains and supplies power at low voltage, by means of a rheostat, to an electric motor designed to move pumping means.

The pumping means are adapted to generate the pulsed flow of the washing liquid.

The pumping means comprise a reciprocating piston pump (generally of the single-cylinder type), which is incorporated within the casing of the irrigator.

The reciprocating pump is generally actuated by an electric motor, which of course is also integrated within the casing of the device.

The electric motor is powered by a transformer instead of batteries as usual in the background art.

Batteries in fact have the advantage of having a low voltage and amperage (higher safety) but have the problem of limited life and of the consequent need to have a plurality of batteries available during surgical operations (or to have a container that contains enough of them to ensure the running of the motor for the entire operation).

Moreover, depending on their charge, the power supply to the motor is never constant and optimum, with repercussions on the pressure of the flow of the pump.

Moreover, these batteries must be arranged in disposable containers which must be sterilized (in addition to having a certain bulk).

Further, the batteries, once their life has ended, require disposal.

These surgical irrigators powered by means of a transformer, despite being adopted successfully, have an aspect that can be improved and is linked to the limit of the electric power supply that the transformer imposes on the electric motor for driving the reciprocating pump.

In some situations the physiological region that is undergoing a surgical operation in fact often requires a more intense and energetic washing than what the surgical irrigator in use can provide.

This entails that often, in order to achieve a certain degree of cleaning, or to remove organic material that is difficult to remove, one resorts to prolonged use, i.e., use which is particularly extended over time, of the irrigator powered at maximum power, with consequent drawbacks linked to the use of large quantities of liquid washing solutions and to the subsequent need for more than one bag for the collection and disposal of the used liquid, in addition of course to the time spent on washing which is taken from the surgical operation.

The aim of the present invention is to provide a surgical irrigator for washing body regions subjected to an operation that solves the drawbacks noted in known types.

Within this aim, an object of the present invention is to provide a surgical irrigator that can ensure a more "energetic" pulsed jet than known similar irrigators and is capable of facilitating the removal of organic material that generally cannot be removed with the cited known irrigators.

Another object of the present invention is to provide a surgical irrigator that can be powered from the mains.

Another object of the invention is to provide a surgical irrigator that can be handled easily by the surgeon.

Another object of the present invention is to provide a surgical irrigator that allows the provision of an optimum pulsed flow even in the steps for starting and stopping the device.

Another object of the present invention is to provide a surgical irrigator that in any case does not increase the quantity of equipment and controls present in the operating region.

Another object of the present invention is to provide a surgical irrigator that can be manufactured with known systems and technologies.

This aim and these and other objects that will become better apparent hereinafter are achieved by a surgical irrigator for washing body regions subjected to an operation, which has a containment casing inside which there are means for pumping a washing liquid that arrives from a tank which can be associated therewith and an electric motor for actuating said pumping means, characterized in that said electric motor is powered by the mains by means of
- a power supply, with which there is associated
- at least one assembly for limiting the output voltage to a value that is not higher than the nominal operating voltage of the electric motor, which in turn comprises
- means for switching the output voltage to a value that is higher than said nominal operating value of the electric motor.

Further characteristics and advantages of the invention will become better apparent from the following detailed description of a preferred but not exclusive embodiment of the surgical irrigator according to the invention, illustrated by way of non-limiting example in the accompanying drawings, wherein:
Figure 1 is a perspective cutout view of an irrigator according to the invention;
Figure 2 is an electrical diagram related to the power supply and to the components associated therewith;
Figures 3 and 4 are two charts of the behavior of the supply voltage of the motor in two different modes.

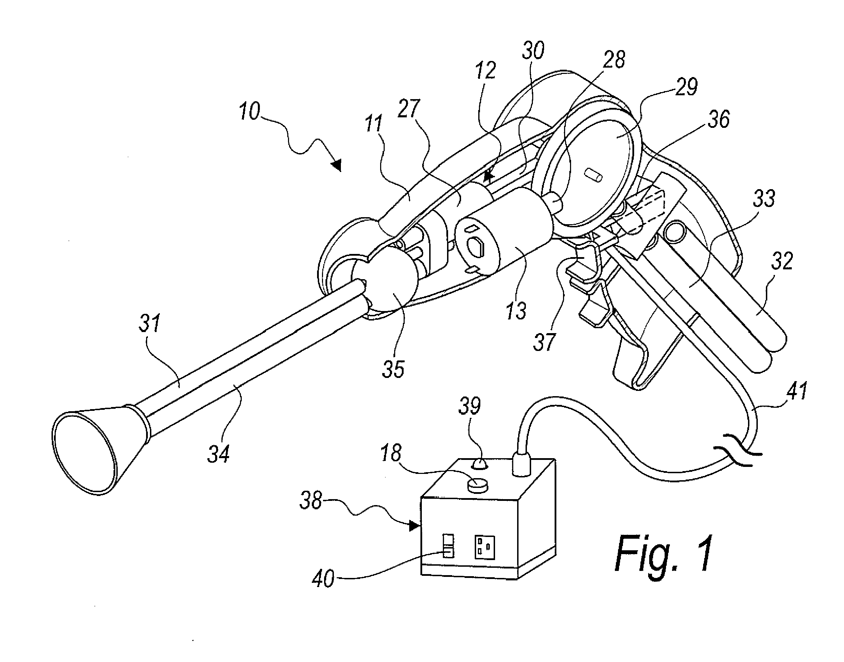

With reference to the figures, a surgical irrigator is designated by the reference numeral 10 in Figure 1.

The surgical irrigator 10, designed to wash body regions subjected to an operation, has a containment casing 11.

Inside the casing 11 there are means 12 for pumping a washing liquid that arrives from a tank which can be associated therewith, and an electric motor 13 for actuating the pumping means 12.

The electric motor 13 is of the direct-current type.

The electric motor 13 is powered by the mains by means of
- a power supply 14, with which, in this exemplifying and non-limiting embodiment of the invention, there are associated
- two assemblies, a first assembly 15 and a second assembly 16
respectively, which limit the output voltage to a value Vu1 (where Vu refers to the output voltage) and Vu2 respectively, which is not higher than the nominal operating value Vn of the electric motor 13.

Means 17 for switching the output voltage Vu1 or Vu2 to a value Vu3 that is higher than the nominal operating value Vn of the electric motor 13 are associated with the two voltage limiting assemblies 15 and 16.

The output voltage switching means 17 comprise, in this exemplifying but non-limiting embodiment of the invention, a manual switch 18 for first activation and a timer assembly 19, which is programmable for stable or temporary deactivation according to time intervals that can be predefined.

In particular, the closure of the switch 18 switches on a transistor 20, for example of the Q1 type, designed to bypass the associated voltage limiting assembly 15 or 16.

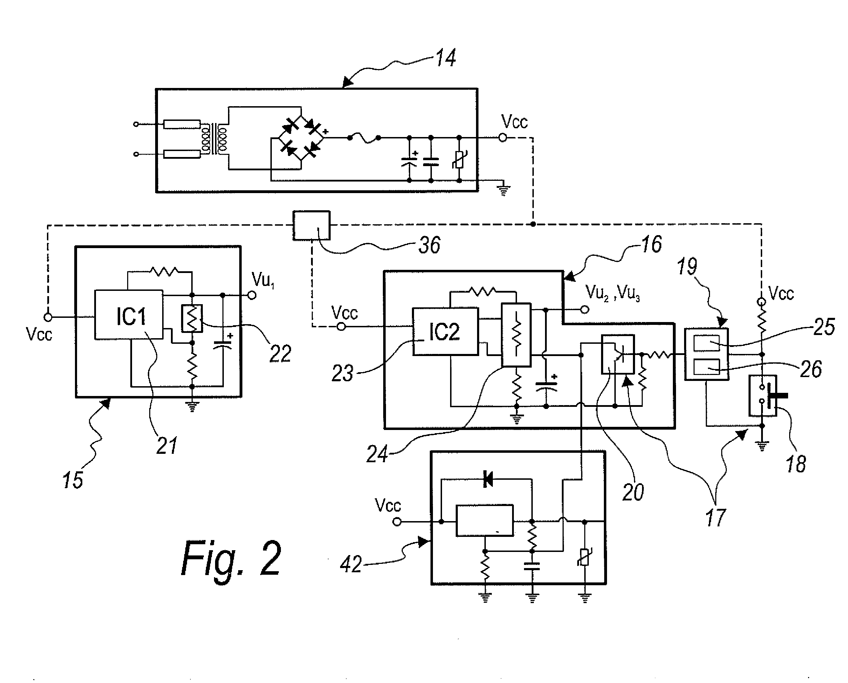

In Figure 2, the voltage switching means 17 are shown schematically associated with the second voltage limiting assembly 16.

The first voltage limiting assembly 15 comprises a first integrated voltage regulator circuit 21, for example of the type known as L200, associated with a first trimmer 22.

The second voltage limiting assembly 16 comprises a second integrated voltage regulator circuit 23, for example also of the type known as L200, which is associated with a second trimmer 24.

The timer assembly 19, of a type known per se and optionally commercially available, comprises a first timer 25, by means of which the time interval DT1 of the operation of the electric motor 13 at the voltage Vu3, higher than the nominal voltage Vn, is set and predetermined.

This time interval DT1 is such as to not entail failure of the electric motor 13.

The timer assembly 19 comprises a second timer 26, also of a type known per se, by means of which the time interval DT2 in which the electric motor 13 is prevented from operating at the voltage Vu3, immediately after its operation at Vu3 has been interrupted, is set and predetermined.

In this second time interval DT2, the electric motor 13 can operate at the voltages Vu1 or Vu2, which are not higher than the nominal voltage Vn.

The time interval DT2 is such as to ensure the restoring of such temperature conditions as to allow the electric motor 13 to resume operation both at the normal voltages Vu1 and Vu2 and at the higher voltage Vu3 without the risk of failures.

The pumping means 12 comprise a reciprocating pump 27, which in the present embodiment is of the single-acting single-cylinder type.

The electric motor 13 moves the reciprocating pump 27 by means of a pinion 28 and a gear 29, to which a linkage 30 is fixed eccentrically which operates on the reciprocating pump 27.

Figure 1 illustrates the ejection duct 31 and the suction duct 32.

The irrigator 10 can also comprise suction means for removal by suction of organic material or liquid in excess on the operated part. These suction means, connected to a suction pump which is external to the casing 11, comprise a flexible hose 33 for connection between the pump and the irrigator 10 and a terminal suction duct 34.

The irrigator 10 described here has a spherical joint which is constituted by the spherical body 35, which is provided with a hole for the passage of the washing liquid and of the aspirated material.

The electric motor 13 is actuated by means of a switch 36, which in the present embodiment has three positions (off, first supply voltage Vu1, second supply voltage Vu2) in which the user acts by means of a button 37.

The switch 36 is also shown schematically in Figure 2.

The power supply 14 and the voltage limiting assemblies 15 and 16, with the output voltage switching means 17, and the timer assembly 19, are contained within a single box-like body 38, as shown in Figure 1.

The button 18 for the manual activation and deactivation of the means 17 for increasing the output voltage, an LED 39 for indicating power-on, and a switch 40 for switching on the power supply 14 protrude from the box-like body 38.

An electrical cable 41 connects the box-like body 38 to the switch 36.

Figure 2 also shows a further block 42 for the continuous adjustment of the output voltage between a minimum voltage and the voltage Vcc at the output of the power supply 14, which also can be associated with the power supply in a further embodiment of the irrigator 10 according to the invention.

Two examples of operation of the irrigator 10 according to the invention are described hereafter.

In a first example of operation, assume that the following are set:
- the power supply 14, to reduce the voltage drawn from the mains, for example 230 V DC, to a voltage Vcc in output from the power supply 14 of 12 volts;
- the first integrated circuit 21 and the first trimmer 22 so that the first output voltage Vu1 is approximately 6 volts;
- the second integrated circuit 23 and the corresponding second trimmer 24 so that the second output voltage Vu2 is approximately 9.5 volts;
- with nominal voltage Vn = 10 volts for the electric motor 13.

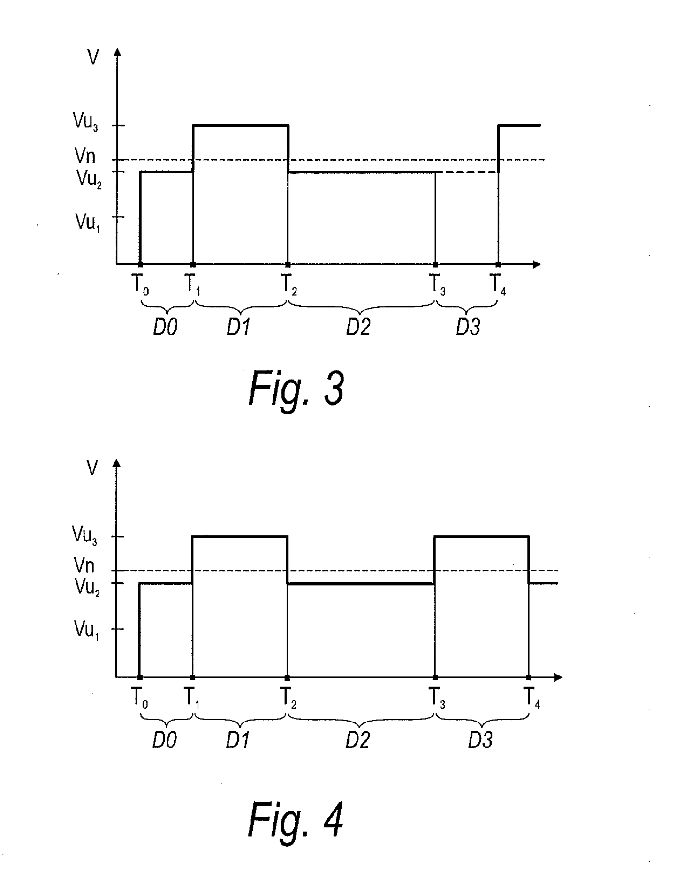

This operation is shown schematically in the chart of Figure 3, where the abscissas plot the time T from the power-on of the electric motor 13 and the ordinates plot the supply voltage V of the electric motor 13.

By oprating the button 37 associated with the casing 11 of the irrigator 10, the user starts the electric motor 13, selecting initially either the first voltage Vu 1 or the second voltage Vu2.

The user, for example, activates the second output voltage Vu2, of 9.5 volts, to perform an energetic washing, which however proves insufficient to achieve the desired cleaning of the part; thus after a first time interval DT0 = T0 - T1, the user (the surgeon), or one of his/her assistants (a nurse), presses the button 18, allowing the supply of the electric motor 13 at the voltage Vu3 equal to the voltage Vcc = 12 volts in output from the power supply 14.

By supplying the electric motor 13 at a voltage Vu3 that is higher than its nominal voltage Vn, one obtains a higher pulsing frequency of the washing flow and a consequent higher flow-rate, which makes it possible to obtain a faster and more energetic cleaning of the part.

The first timer 25 is designed, after a first safety time interval D1, to open the circuit, which is closed by the first pressing of the button 18, thus interrupting the operation of the electric motor 13 at 12 volts, a voltage which is higher than the nominal voltage Vn = 10 volts.

The safety interval D1 is such as to prevent operation at 12 volts from continuing for such a time as to damage the electric motor 13 or the associated reciprocating pump 27 (gaskets or other components) or the intermediate gears.

For example, D1 is set equal to 7 seconds.

The chart of Figure 3 thus shows that after seven seconds the supply voltage of the electric motor 13 drops again to Vu2 = 9.5 volts.

At this point the second timer 26, designed to prevent, for a certain time interval D2, the restarting of operation at the higher voltage Vu3 = Vcc = 12 volts, starts to operate.

The waiting time interval D2 is such as to ensure cooling of the electric motor 13 after operation at a voltage that is higher than the nominal one, and of any components of the pump 27 that might likewise be heated inappropriately.

Assume D 1 is, for example, an interval of 10 seconds.

Within this interval D2, if the user again pressed the button 18 for starting the operation at the higher voltage Vu3, he would achieve no effect.

Once the second interval D2 has elapsed, the user, by pressing again at a certain point in time T4 the button 18, after a certain time interval D3, restarts operation at the higher voltage Vu3 for a further time interval D1 of seven seconds, followed by a new waiting interval D2.

This sequence of operations continues at the user's discretion.

In a second example of operation of the irrigator 10 according to the invention, the electric motor 13 is started initially by selection of a second output voltage Vu2 at 9.5 volts (but likewise also for Vu1 = 6 volts) and after a first time interval DT0 = T0 - T1 the button 18 is pressed, allowing the supply of the electric motor 13 at the voltage Vu3 equal to the voltage Vcc = 12 volts in output from the power supply 14.

The first timer 25 is designed, after a first safety time interval D1, to interrupt the operation of the electric motor 13 at 12 volts.

Again, D 1 is set equal to seven seconds.

For these seven seconds, the supply voltage of the electric motor 13 remains at Vu3 = Vcc = 12 volts; at the end of such seven seconds the voltage returns to Vu2 = 9.5 volts.

At this point the second timer 26 starts and the waiting interval D2, set for example to 10 seconds, begins.

If the button 37 associated with the handpiece of the irrigator 10 has been kept pressed during this waiting interval D2, or if it has been pressed again during the interval D2, at the end of the interval D2 operation at the higher voltage Vu3 = Vcc = 12 volts resumes for another time interval D3 of seven seconds, followed by another waiting interval of ten seconds, and so forth, until the button 18 on the box-like body 38 is pressed to interrupt permanently operation at the higher voltage Vu3.

In practice it has been found that the invention achieves the intended aim and objects.

In particular, the invention provides a surgical irrigator that is capable of ensuring a more "energetic" pulsed jet than similar known irrigators and is capable of facilitating the removal of organic material that generally cannot be removed with the above cited known irrigators.

Further, the present invention provides a surgical irrigator that can be powered by electrical outlets of a known type of a standard electrical grid, such as a national grid, as well as by an electrical system of an emergency vehicle or any domestic outlet.

Moreover, the present invention provides a surgical irrigator that can be handled easily by the surgeon in the same way as known irrigators.

Moreover, the present invention provides a surgical irrigator that allows the provision of an optimum pulsed flow even during the starting and stopping steps of such device.

Further, thanks to the compactness of the box-like body that contains the power supply, the voltage limiting assemblies and the timer assembly, the present invention provides a surgical irrigator that in any case does not increase the quantity of equipment and controls that are present in the operation region or the bulks that cause hindrance in an operating room.

Moreover, the present invention provides a surgical irrigator that can be manufactured with known systems and technologies.

The invention thus conceived is susceptible of numerous modifications and variations, all of which are within the scope of the appended claims; all the details may further be replaced with other technically equivalent elements.

In practice, the materials used, as well as the contingent shapes and dimensions, may be any according to requirements and to the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A surgical irrigator (10) for washing body regions subjected to an operation, which has a containment casing (11) inside which there are means (12) for pumping a washing liquid that arrives from a tank which can be associated therewith and an electric motor (13) for actuating said pumping means, **characterized in that** said electric motor (13) is powered by the mains by means of
- a power supply (14), with which there is associated
- at least one assembly (15, 16) for limiting the output voltage to a value (Vu1, Vu2) that is not higher than the nominal operating voltage (Vn) of the electric motor (13), with which at least one assembly (16) means (17) are associated for switching the output voltage to a value (Vu3) that is higher than said nominal operating value (Vn) of the electric motor (13).

2. The irrigator according to claim 1, **characterized in that** said means (17) for switching the output voltage comprise a manual switch (18) for first activation and a programmable timer assembly (19) for permanent or temporary deactivation depending on time intervals that can be predefined.

3. The irrigator according to the preceding claims, **characterized in that** the closure of said switch (18) causes the opening of a transistor (20) designed to bypass said associated voltage limiting assembly (15, 16).

4. The irrigator according to the preceding claims, **characterized in that** it comprises two voltage limiting assemblies (15, 16), a first voltage limiting assembly (15) which comprises a first integrated voltage regulator circuit (21), for example of the type known as L200, associated with a first trimmer (22), and a second voltage limiting assembly (16), which comprises a second integrated voltage regulator circuit (23), for example also of the type known as L200, associated with a second trimmer (24).

5. The irrigator according to the preceding claims, **characterized in that** said timer assembly (19) comprises
- a first timer (25), by means of which a first time interval (DT1) is set and predetermined which is related to the operation of the electric motor (13) at the voltage (Vu3), which is higher than the nominal voltage (Vn),
- a second timer (26), by means of which a second time interval (DT2) is set and predetermined in which the electric motor (13) is prevented from working at the higher voltage (Vu3) immediately after its operation at said higher voltage (Vu3) has been interrupted.

6. The irrigator according to the preceding claims, **characterized in that** said pumping means (12) comprise a reciprocating pump (27).

7. The irrigator according to the preceding claims, **characterized in that** the actuation of the electric motor (13) is performed by means of a switch (36), which the user operates by means of a button (37).

8. The irrigator according to the preceding claims, **characterized in that** the power supply (14) and the voltage limiting assemblies (15, 16), with the means (17) for switching the output voltage, and the timer assembly (19), are contained within a single box-like body (38), the button (18) for manual activation and deactivation of the means (17) for increasing the output voltage and a power-on indication LED (39) protruding from said box-like body (38).

## Amended claims

### Amended claims in accordance with Rule 137(2) EPC.

**1.** A surgical irrigator (10) for washing body regions subjected to an operation, which has a containment casing (11) inside which there are means (12) for pumping a washing liquid that arrives from a tank which can be associated therewith and an electric motor (13) having a nominal operating voltage (Vn) for actuating said pumping means, said electric motor (13) being powered by the mains by means of
- a power supply (14), with which there is associated
- at least one assembly (15, 16) for setting the output voltage supplied to said electric motor (13) to a value (Vu1, Vu2) that is not higher than the nominal operating voltage (Vn) of the electric motor (13),
**characterized in that** means (17) are associated with said at least one assembly (16) for switching the output voltage supplied to said electric motor (13) to a value (Vu3) that is higher than said nominal operating value (Vn) of the electric motor (13), said means (17) for switching the output voltage supplied to said electric motor (13) comprise a manual switch (18) for first activation and a programmable timer assembly (19) for permanent or temporary deactivation depending on time intervals that can be predefined.

**2.** The irrigator according to claim 1, **characterized in that** the closure of said switch (18) causes the opening of a transistor (20) designed to bypass said associated voltage limiting assembly (15, 16).

**3.** The irrigator according to the preceding claims, **characterized in that** it comprises two voltage setting assemblies (15, 16), a first voltage setting assembly (15) which comprises a first integrated voltage regulator circuit (21) associated with a first trimmer (22), and a second voltage setting assembly (16), which comprises a second integrated voltage regulator circuit (23) associated with a second trimmer (24).

**4.** The irrigator according to the preceding claims, **characterized in that** said timer assembly (19) comprises
- a first timer (25), by means of which a first time interval (DT1) is set and predetermined which is related to the operation of the electric motor (13) at the voltage (Vu3), which is higher than the nominal voltage (Vn),
- a second timer (26), by means of which a second time interval (DT2) is set and predetermined in which the electric motor (13) is prevented from working at the higher voltage (Vu3) immediately after its operation at said higher voltage (Vu3) has been interrupted.

**5.** The irrigator according to the preceding claims, **characterized in that** said pumping means (12) comprise a reciprocating pump (27).

**6.** The irrigator according to the preceding claims, **characterized in that** the actuation of the electric motor (13) is performed by means of a switch (36), which the user operates by means of a button (37).

**7.** The irrigator according to the preceding claims, **characterized in that** the power supply (14) and the voltage setting assemblies (15, 16), with the means (17) for switching the output voltage, and the timer assembly (19), are contained within a single box-like body (38), the button (18) for manual activation and deactivation of the means (17) for increasing the output voltage and a power-on indication LED (39) protruding from said box-like body (38).
